# EUROPEAN PATENT SPECIFICATION

(11) **EP 0 540 588 B1**
(45) Date of publication and mention of the grant of the patent: **21.06.1995**
(21) Application number: 91913278.7
(22) Date of filing: 24.07.1991
(51) Int. Cl.: A61K 39/00, A61K 47/48, A61K 49/00

(54) **A COMPOSITION PROVIDING IMPROVED CLEARANCE OF BIOACTIVE SUBSTANCES FROM THE BLOODSTREAM**
EINE ZUSAMMENSETZUNG DIE DIE VERBESSERTE AUSSCHEIDUNG VON BIOAKTIVEN SUBSTANZEN AUS DEM BLUTSTROM ERLAUBT
COMPOSITION ASSURANT UNE MEILLEURE ELIMINATION DE SUBSTANCES BIOACTIVES CONTENUES DANS LE SYSTEME SANGUIN

(30) Priority: 24.07.1990 DK 1762/90
(43) Date of publication of application: 12.05.1993
(73) Proprietor: NOVO NORDISK A/S, 2880 Bagsvaerd (DK)
(72) Inventor: SELMER, Johan, DK-3520 Farum (DK)
(86) International application number: DK9100215
(87) International publication number: WO9201469

(56) References cited:
- EP-A- 0 149 709
- EP-A- 0 187 658
- EP-A- 0 308 208
- EP-A- 0 353 960
- WO-A-87/06263
- WO-A-89/05140
- US-A- 4 624 846
- US-A- 4 863 713
- US-A- 4 932 412
- PATENT ABSTRACTS OF JAPAN, Vol. 7, No. 210, P223, Abstract of JP 58103664, publ 1983-06-20 Teijin K.K.
- Biochemical Society Transactions, Vol. 18, 1990, K.D. BAGSHAWE: "Antibody-directed enzyme/prodrug therapy (ADEPT)", see page 750 - page 752, see page 751 left column "A three-stage system", and "Discussion".
- Science, Vol. 243, June 1989, E HABER et al.: "Innovative Approaches to Plasminogen Activator Therapy", see page 51 - page 56 see pages 55-56.

## Description

### FIELD OF INVENTION

The present invention relates to a pharmaceutical or diagnostic composition which, when administered to a subject, is capable of providing an improved clearance of a bioactive substance from the bloodstream. The invention further relates to methods of providing such improved clearance for diagnostic or therapeutic purposes.

### BACKGROUND OF THE INVENTION

It is known to use antibodies or conjugates of an antibody and a diagnostically or therapeutically active agent to neutralize a biologically active substance, such as a toxin (e.g. lipopolysaccharide; cf., for instance, S.S. Martiny et al., Crit. Care Med. 16, 1988, pp. 629-635), or to diagnose a specific condition in vivo (cf., for instance, J.F. Chatal, Monoclonal Antibodies in Immunoscintigraphy, CRC Press, Florida, USA, 1990), or to target a drug to cells expressing a specific antigen, e.g. tumour cells (cf., for instance, US 4,357,273, EP 336 405 or EP 269 188). Such use of antibodies for diagnostic purposes suffers from the drawback that the rate of clearance or elimination of the antibody (conjugate) from the bloodstream is generally rather slow so that, for an appreciable period of time, the background activity from the circulating diagnostic agent will be too high to permit an accurate diagnosis of the condition in question. This, of course, is a particular disadvantage in case of an acute condition requiring immediate diagnosis and treatment. Similarly, when antibodies or antibody conjugates are administered for therapeutic purposes, e.g. to neutralize a toxin or a drug, it would be a considerable advantage to eliminate the active substance from the circulation fairly rapidly in cases where the neutralization of the active substance is less than optimal (this has been described for lipopolysaccharide, cf. L.S. Young et al., Rev. Infect. Dis. 11, Suppl. 7, 1989, pp. S1564-S1571, or B.J. Appelmelk et al., Miccrob. Pathogen. 5, 1988, pp. 251-257).

In EP 308 208, it is suggested to use a conjugate of an antibody which has been modified so that it is able to bind to a marker produced by a tumour or infectious lesion and a glycoside residue capable of binding to the hepatocyte asialoglycoprotein receptor for the diagnosis or treatment of tumours and infectious lesions. The clearance of the conjugate from the circulation may be deferred by continuously infusing an inhibitor which, by binding to the same receptor, blocks binding of the conjugate.

The method for providing enhanced clearance suggested in EP 308 208 suffers from the drawback that, even when infusion of the inhibitor has ceased, it takes some time before it no longer has any effect, i.e. before the hepatocyte receptor is no longer blocked. This makes the conjugate less favourable for use in emergency situations where rapid diagnosis and treatment is essential. Furthermore, it would not be convenient for patients to whom the conjugate is administered to be subjected to continuous infusion of the inhibitor for a period of time of up to several days (this is particularly relevant in case of tumour diagnosis and/or treatment).

E. Haber et al, Science 243, 1989, pp. 51-56, describe a conjugate of t-PA and an anti-fibrin antibody for the localization of t-PA in the vicinity of fibrin deposits. The conjugate is suggested for use as an antithrombotic agent. The possibility of using t-PA as a ligand to eliminate the conjugate from the circulation is not mentioned in the article.

In US 4,863,713, a binding protein which binds to a tumour antigen is administered and circulating binding protein is eliminated by administering a clearing agent after which an epitopic compound which binds to the binding protein is administered. The epitopic compound acts as a kind of label, and it is quickly eliminated from the circulation if not bound to the binding protein.

WO 89/05140 describes a tumour targeting agent composed of a Fab fragment and a ligand which binds to a binding protein. The ligand does not bind to a receptor causing the agent to be cleared from the circulation. Clearance may be provided by means of a substance competing with the tumour targeting agent for binding to the binding protein.

JP 58-103664 describes a selective cell killing method by means of an aantitumou agent composed of a biotinylated tumour-specific antibody, a biotinylated cytotoxic substance, and avidin. There is no suggestion of clearance of this agent from the circulation.

WO 87/06263 describes a fibrin-specific monoclonal antibody for detection of thrombi as well as a thrombolytic agent composed of the antibody coupled to a thrombolytic agent, e.g. t-PA or urokinase. There is no indication of clearance of the thrombolytic agent from the circulation.

The object of the present invention is to provide an improved composition and method for providing a faster rate of clearance of biologically active substances from the circulation.

### SUMMARY OF THE INVENTION

Accordingly, the present invention relates to a pharmaceutical or diagnostic composition comprising, in separate containers,
(a) a capturing agent capable of binding to a bioactive substance so as to make it possible to locate, neutralize and/or remove the bioactive substance on administration of the capturing agent; and
(b) a ligand or ligand analogue capable of binding to a cellular receptor found in an organ through which waste products of the body are usually eliminated,
   the capturing agent being provided with means to bind the ligand or ligand analogue, or the ligand or ligand analogue being provided with means to bind the capturing agent, or both the capturing agent and the ligand being provided with complementary binding means.

In the present context, the term "bioactive substance" is used to indicate any substance which has a biological function or exerts a biological effect in the human or animal body or is formed in the human or animal body in response to a biological process (which might, for instance, be a pathological process) or is released to the circulation in response to a biological process. The term "ligand" is intended to indicate a substance which, in nature, is capable of binding to a particular cellular receptor, while the term "ligand analogue" is used to indicate a substance which is similarly capable of binding to the same cellular receptor. The ligand analogue may be a derivative of the native ligand which has retained its ability to bind to the cellular receptor in question.

The term "cellular receptor" is intended to indicate a receptor located on cell surfaces and comprising an extracellular domain with ligand-binding properties. Some receptors have a transmembrane domain which anchors the receptor in the cell membrane and a cytoplasmic domain which generates a cellular signal in response to ligand binding. Other receptors may be anchored to the cell membrane by a phospholipid chain or indirectly by non-covalent binding to transmembrane proteins. Still other receptors are glycolipids which are anchored in the cell membrane by their lipid chains.

In the process of receptor-mediated endocytosis, ligand binding to receptors induces the collection of ligand-receptor complexes in coated pits on the cell surface. These subsequently invaginate to form coated vesicles which fuse with lysosomes followed by enzymatic transformation of the contents of the vesicles (cf. I.H. Pastan and M.C. Willingham, Ann. Rev. Physiol. 43, 1981, pp. 239-250). Endocytotic vesicles may also form from uncoated regions on the plasma membrane (cf. B. Alberts et al., The Molecular Biology of the Cell, Gerland Publ. Inc., New York & London, 1983). Furthermore, phagocytosis may be induced by binding to specific receptors (cf. Alberts et al, op. cit.). Insoluble immunocomplexes may thus be taken up by Kupffer cells via IgG-Fc receptors (cf. B. Smedsrød et al., Biochem. J. 266, 1990, pp. 313-327; S.M. Van der Laan-Klamer et al., Scand. J. Immunol. 23, 1986, pp. 441-447).

The composition of the invention may be used in a method of providing the rapid clearance of a bioactive substance from the circulation, the method comprising administering, to a subject in need hereof,
(a) an effective amount of a capturing agent capable of binding to a bioactive substance so as to make it possible to locate, neutralize and/or remove the bioactive substance on administration of the capturing agent,
   followed, after a suitable interval, by administration of
(b) an effective amount of a ligand or ligand analogue capable of binding to a cellular receptor found in an organ through which waste products of the body are usually eliminated,
   the capturing agent being provided with means to bind the ligand or ligand analogue, or the ligand or ligand analogue being provided with means to bind the capturing agent, or both the capturing agent and the ligand being provided with complementary binding means.

The present method has the advantage that the interval with which the capturing agent and subsequently the ligand or ligand analogue is administered may be varied at will according to the time it is estimated to take for the capturing agent to bind a sufficient amount of the bioactive substance. Likewise, the method is flexible with respect to the receptor cell through which the bioactive substance is eliminated, making it possible to avoid overloading a particular organ through which the bioactive substance would normally be excreted, e.g. the kidneys. Furthermore, patients receiving this treatment are not significantly inconvenienced by being subjected to the method.

It should be noted that the bioactive substance need not in each instance be eliminated from the body through receptor binding and cellular uptake. Rather, the cells may also act as a storage facility for the bioactive substance resulting in a protracted release of the bioactive substance to the bloodstream. In this way, the acute toxicity to the excreting organ (e.g. the kidneys) or normally metabolizing organ (e.g. the thyroid gland) may be significantly reduced.

The composition of the invention may furthermore be used in a method of rapid in vivo detection of a pathological condition, the method comprising administering
(a) an effective amount of a capturing agent capable of binding to a bioactive substance characteristic of the pathological condition in question so as to make it possible to locate, neutralize and/or remove the bioactive substance on administration of the capturing agent,
   followed, after a suitable interval, by administration of
(b) an effective amount of a ligand or ligand analogue capable of binding to a cellular receptor found in an organ through which waste products of the body are usually eliminated,
   the capturing agent being provided with means to bind the ligand or ligand analogue, or the ligand or ligand analogue being provided with means to bind the capturing agent, or both the capturing agent and the ligand being provided with complementary binding means, so as to provide a rapid clearance of the capturing agent from the circulation.

After the capturing agent has been cleared from the bloodstream, the visualization of any capturing agent binding to the bioactive substance may be carried out by any suitable means of conducting in vivo diagnosis. The advantage of the present method is that interference from background activity caused by circulating capturing agent may be substantially eliminated within a short period of time, making the method particularly useful for diagnosing conditions where rapid treatment is essential.

US 4,624,846 discloses a method of localizing a tumour by injecting a radiolabelled antibody against a tumour marker followed by injection, after a suitable interval permitting uptake of the antibody by the tumour, of an antibody against the radiolabelled antibody whereby the level of circulating radiolabelled antibody decreases by 10-85% in 2-72 hours. This rate of clearance is rather slow compared to that obtainable with the composition of the present invention, making the method of US 4,624,846 less suitable for rapid diagnosis. A similar method is described in EP 353 960.

US 4,634,586 discloses a method of radioimaging leukocytes by injecting a conjugate of an antibody reactive with a leukocyte surface molecule and a radioisotope chelated with an EDTA or DTPA derivative followed by the injection of an antibody against the conjugate in order to clear the conjugate/antibody complex through the reticuloendothelial system. In a dog study, a large amount of anti-conjugate antibody injected after injection of the conjugate is reported to result in clearance of 90% of the conjugate within 15 minutes. It should, however, be noted that elimination from dog circulation is, at any rate, faster than elimination from human circulation. Furthermore, it should be emphasized that the antibody conjugated with the radioisotope must be foreign to the species into which it is injected as the subsequently injected anti-conjugate antibody would otherwise not react specifically with the conjugate. This is not the case with the present composition and method, making it possible to utilize human (or humanized) substances as the capturing agent and ligand, thereby significantly reducing the risk of adverse immune reactions.

In a further aspect, the present invention relates to the use of a capturing agent and a ligand or ligand analogue for the manufacture of a pharmaceutical composition providing the rapid clearance of a bioactive substance from the circulation and comprising
(a) a capturing agent capable of binding to a bioactive substance so as to make it possible to locate, neutralize and/or remove the bioactive substance on administration of the capturing agent; and
(b) a ligand or ligand analogue capable of binding to a cellular receptor found in an organ through which waste products of the body are usually eliminated,
   the capturing agent being provided with means to bind the ligand or ligand analogue, or the ligand or ligand analogue being provided with means to bind the capturing agent, or both the capturing agent and the ligand or ligand analogue being provided with complementary binding means,
   the composition being adapted for separate and sequential administration of the capturing agent and the ligand or ligand analogue.

In a still further aspect, the present invention relates to the use of a capturing agent and a ligand or ligand analogue for the manufacture of a diagnostic composition providing the rapid clearance of the capturing agent from the circulation and hence a rapid detection of the pathological condition in question, the composition comprising
(a) a capturing agent capable of binding to a bioactive substance so as to make it possible to locate, neutralize and/or remove the bioactive substance on administration of the capturing agent; and
(b) a ligand or ligand analogue capable of binding to a cellular receptor found in an organ through which waste products of the body are usually eliminated,
   the capturing agent being provided with means to bind the ligand or ligand analogue, or the ligand or ligand analogue being provided with means to bind the capturing agent, or both the capturing agent and the ligand or ligand analogue being provided with complementary binding means,
   the composition being adapted for separate and sequential administration of the capturing agent and the ligand or ligand analogue.

### DETAILED DISCLOSURE OF THE INVENTION

In one embodiment of the present invention, it is desired to clear the capturing agent rapidly from the circulation. This is particularly the case when the present composition is used for diagnostic purposes wherein, once the capturing agent is bound to the bioactive substance, it is a considerable advantage to eliminate any capturing agent remaining in the bloodstream quickly in order to avoid excessive background interference.

In this embodiment, the capturing agent may be monofunctional which is to say that it is able to bind to one substance only. In order for the diagnostic method of the invention to be operable, the ligand or ligand analogue may have to be modified so as to be able to bind to the capturing agent as well as to the receptor. Alternatively, the bioactive substance and the ligand may be substantially identical. In the latter case, administration of the bioactive substance, which when it circulates will act as a ligand binding to a specific cellular receptor, subsequently to the administration of the capturing agent will result in the clearance of unbound capturing agent from the circulation. Apart from this, the capturing agent may be bifunctional which is understood to mean that it is capable of binding to two different substances (typically the bioactive substance and the ligand or ligand analogue which will be different). Also when the capturing agent is bifunctional, the ligand may be modified so as to be able to bind to the capturing agent.

In another embodiment of the present invention, it is desired to clear the bioactive substance rapidly from the circulation. This is particularly the case when the present composition is used for therapeutic purposes where binding to the capturing agent does not neutralize all the biological activity of the bioactive substance or where the binding affinity of the capturing agent is not high enough to reduce the free concentration of the bioactive substance to any significant extent. In such a case clearance of the bound bioactive substance from the bloodstream will be important.

In this embodiment, the capturing agent may be monofunctional (as defined above), in which case the ligand or ligand analogue will have to be modified so that it is able to bind both the capturing agent and the specific cellular receptor. The bioactive substance will be different from the ligand or ligand analogue. Alternatively, the capturing agent may be bifunctional (as defined above), in which case the the bioactive substance and the ligand or ligand analogue will typically be different. Also when the capturing agent is bifunctional, the ligand may be modified so as to be able to bind to the capturing agent.

In case the capturing agent is monofunctional, it may comprise an antibody which is reactive with the bioactive substance, or a cellular receptor (other than the specific ligand-binding receptor) or a fragment thereof including the binding site for the bioactive substance. The ligand or ligand analogue may then be modified so as to comprise an antibody (including an anti-idiotype antibody), a cellular receptor (other than the specific ligand-binding receptor) or a fragment thereof including the binding site for the capturing agent, or wherein the ligand or ligand analogue is modified with a bioactive substance or a derivative or fragment thereof capable of binding the capturing agent.

In the present context, the term "antibody" is used to indicate any substance formed in the human or animal body or by a human or animal cell as a response to exposure to an antigen. The antibody may be a polyclocal or monoclonal antibody or may be one prepared by recombinant DNA techniques. The term also includes antibody fragments, such as Fab', F(ab')₂ or Fv fragments as well as single-domain antibodies.

Monoclonal antibodies may be obtained by well-established methods, e.g. as described in A. Johnstone and R. Thorpe, Immunochemistry in Practice, 2nd. Ed., Blackwell Scientific Publications, 1987, pp. 35-43. When prepared by recombinant DNA techniques, the antibody may be produced by cloning a DNA sequence coding for the antibody or a fragment thereof into a suitable cell, e.g. a microbial, plant, animal or human cell, and culturing the cell under conditions conducive to the production of the antibody or fragment in question and recovering the antibody or fragment thereof from the culture. Possible strategies for the preparation of cloned antibodies are discussed in, for instance, L. Riechmann et al., Nature 332, 24 March 1988, p. 323 ff., describing the preparation of chimeric antibodies of rat variable regions and human constant regions; M. Better et al., Science 240, 20 May 1988, p. 1041 ff., describing the preparation of chimeric mouse-human Fab fragments; A. Sharra and A. Plückthun, Science 240, 20 May 1988, pp. 1038-1040, describing the cloning of an immunoglobulin Fv fragment containing antigen-binding variable domains; and E.S. Ward et al., Nature 341, 12 October 1989, pp. 544-546, describing the cloning of isolated antigen-binding variable domains ("single-domain antibodies").

The term "anti-idiotype antibody" refers to an antibody which is reactive with the epitope binding site of another antibody. The anti-idiotype antibody may be prepared by any one of the methods indicated above.

In certain cases where the ligand or ligand analogue is modified with a bioactive substance, it may be necessary to modify the bioactive substance in order to reduce the risk of adverse effects arising from the administration of the modified ligand. Thus, if the bioactive substance is one which, in the unmodified state, would be toxic to the human or animal body, one may suitably employ a detoxified derivative thereof, e.g. a detoxified fragment thereof. It may, in any case, be preferred to use fragments of the bioactive substance as this will reduce the size of the modified ligand molecule.

When the capturing agent in the composition of the invention is bifunctional, it may suitably comprise a bispecific antibody, i.e. an antibody which is reactive with two different antigens or epitopes. The bispecific antibody may, for instance, be synthesized chemically by cross-linking antibodies of different specificities or by combining one antibody fragment containing at least one epitope-binding site responsible for the reactivity of the antibody with the bioactive substance with another antibody fragment containing at least one epitope-binding site responsible for the reactivity of the antibody with the ligand or ligand analogue, to form a hybrid antibody (cf., e.g., EP 179 872). Alternatively, bispecific antibodies may be formed by fusing two or more hybridomas, e.g. polydomas (cf., for instance, NL 86-1542, EP 68 763 or EP 338 497), or by recombinant DNA techniques (e.g. as described by W.D. Huse et al., Science 246, 1989, pp. 1275-1281 or E.S. Ward et al., Nature 341, 1989, pp. 544-546).

The bifunctional capturing agent may also comprise
a conjugate of an antibody and a cellular receptor (other than the specific ligand-binding receptor) or a fragment thereof comprising the binding site for the bioactive substance, a single-stranded oligonucleotide, an intercellular adhesion molecule, or a complexing agent (e.g. biotin, avidin, lectin or a drug), or
a conjugate of a cellular receptor (other than the specific ligand-binding receptor) or a fragment thereof comprising the binding site for the bioactive substance and a single-stranded oligonucleotide, an intercellular adhesion molecule, or a complexing agent (e.g. biotin, avidin, lectin or a drug).

In this case, too, the ligand or ligand analogue may be modified so as to comprise an antibody, a cellular receptor (other than the ligand-binding receptor) or a fragment thereof comprising a binding site for the capturing agent, a single-stranded oligonucleotide, an intercellular adhesion molecule or a complexing agent, each of which is capable of forming a conjugate with the part of the capturing agent which does not bind the bioactive substance.

Thus, when the capturing agent comprises a single-stranded oligonucleotide, the ligand or ligand analogue will typically be modified with a complementary single-stranded oligonucleotide. Likewise, when the capturing agent comprises an intercellular adhesion molecule or a complexing agent, the ligand or ligand analogue will usually be modified with a substance capable of binding the intercellular adhesion molecule and complexing agent (e.g. avidin or streptavidin if the capturing agent is biotin, biotin if the capturing agent is avidin, a carbohydrate if the capturing agent is a lectin, etc.), respectively. Suitable lectins may be derived from plant (cf. H. Lis and N. Sharon, Ann. Rev. Biochem. 42, 1973, pp. 541-551) or mammalian sources (cf. B. Smedsrød et al., Biochem. J. 266, 1990, pp. 313-327, or Magnusson et al., Biochem. J. 257, 1989, pp. 651-656).

For diagnostic purposes, the capturing agent may suitably be provided with a label. The substance used to label the capturing agent may for instance be a radioactive isotope such as In-111, Tc-99m, I-131, I-125 or I-123 (cf. US 4,357,273, EP 336 405 or EP EP 269 188). Alternatively, the capturing agent may be labelled with a substance useful for magnetic resonance imaging. Examples of such substances are magnetite particles which may be coated with the antibody and used for imaging substantially as described in S. Cerdan et al., Magnetic Resonance in Medicine 12, 1989, pp. 151-163, or paramagnetic atoms, e.g. C¹³ or gadolinium (cf., for instance, J.C. Saccavini et al., Invest. Radiol., Suppl. 1, 1989, pp. S292-S293, or P. Shrere and A. M. Aisen, Magnetic Resonance in Medicine 3, 1986, pp. 336-340).

As indicated above, the cellular receptor to which the ligand or ligand analogue binds after binding to the capturing agent is a receptor which is found in an organ through which waste products of the body are usually eliminated, e.g. the liver, the spleen, the bone marrow, the lungs, the kidneys, the skin or the placenta.
The liver has an important function as a scavenger in the body (cf. B. Smedsrød et al., op. cit.). In the liver, the population of endothelial cells and Kupffer cells compose the hepatic reticuloendothelial system which is responsible for the uptake of various macromolecules, including nanoparticles and particulate matter, respectively (cf. E. Wisse et al. (Eds.), Cells of the Hepatic Sinusoid, Vol 2, The Kupffer cell Foundation, Rijswijk, The Netherlands, 1989). Receptors responsible for the binding of such molecules include, i.a., the hyaluronan, collagen, PIIINP (N-terminal propeptide of collagen type III), mannose/N-acetylglucosamine, complement, asialoglycoprotein and IgG Fc receptors (cf. B. Smedsrød et al., op. cit.). Binding of ligands to these receptors results in receptor-mediated endocytosis whereby vesicles containing the ligand-receptor complex are fused with lysosomes and subsequently degraded. Transcytosis to the parenchymal cells is another possibility (cf. B. Smedsrød et al., op. cit.) as is direct phagocytosis of the Kupffer cells which sequester particulate matter. A further suitable receptor is the hepatic receptor for tissue plasminogen activator.

Identical or similar reticuloendothelial cells may also exist in other organs such as the spleen, the lungs, the bone marrow or the placenta. The cells in these organs may be equipped with special receptors characteristic of the organs.

Other types of receptors include receptors for peptide or polypeptide hormones and growth factors shown in Table I below.

**Table I**

| | |
|---|---|
| Receptors for growth factors | epidermal growth factor, transforming growth factor, insulin-like growth factor, platelet-derived growth factor, interleukin-1α, interleukin-1β, interleukin-2, colony stimulating factor, γ-interferon, nerve growth factors, tumour necrosis factor |
| Receptors for peptide hormones | T3, T4, oxytocin, histamine, vasopressin |
| Receptors for polypeptide hormones | growth hormone, insulin, glucagon, thyroid-stimulating hormone, follicle-stimulating hormone, luteinising hormone, adrenocorticotropic hormone, parathyroidea hormone, prolactin, lipotropin, cholecystokinin, calcitonin, secretin, atrialnatriuretic factor, endothelin, vasoactive intestinal polypeptide, transferrin, tachykinin |
| Intercellular adhesion factors | intercellular adhesion molecule 1, endothelial leukocyte adhesion molecule 1, vascular cell adhesion molecule 1 |

When the corresponding ligands bind to these receptors, an intracellular signal is triggered in response. When such receptors are used for the elimination of the bioactive substance, care should be taken that the binding of the conjugate of the bioactive substance, capturing agent and ligand to the receptor should not trigger an intracellular signal to such a degree that it has any significant overall physiological impact.

Another group of receptors are those located intracellularly which mainly bind hydrophobic ligands. Examples of such receptors are shown in Table II below

**Table II**

| | |
|---|---|
| Receptors for vitamins | Vitamin A, Vitamin D, retinoic acid |
| Receptors for fatty acid derivatives | prostaglandin B1, D2, E1, E2, F2, leukotriene, thromboxane B2 |
| Receptors for steroid hormones | testosterone, progesterone, cortisol, estradiol, aldosterone |

A third group of receptors are those which are located on cells of the nervous system, e.g. α-adrenergic, β-adrenergic, epinephrine, adrenalin, dopamin, opiate (such as morphine, enkephaline, endorphin) angiotensin, adenosin, benzodiazepine, bradykinin, choline muscarinic, cholinergic nicotinic, GABA or serotonin receptors. These receptors may, in certain cases, be used for the elimination of the bioactive substance provided that they are sufficiently abundant and not protected by the blood-brain barrier. In this case, too, it is important that the physiological receptor response does not have any significant overall impact on the body.

The spleen forms part of the reticuloendothelial clearance syrtem and as such includes similar cells as those described above for the liver sinusoids. However, the spleen may also contain specific receptors which are different from those of the liver. Apart from this general clearance function, the spleen sequestrates aged erythrocytes. The signal for the retention of erythrocytes in the spleen is not known (M.R. Clark, Physiol. Rev. 68, 1988, pp. 503-554), but the presence of a senescent antigen derived from the band 3 protein in combination with auto-antibodies against this antigen may be the signal that triggers the sequestration, e.g. via the Fc receptors (M.M.B. Kay, Proc. Natl. Acad. Sci. USA 81, 1984, pp. 5753-5757).

Apart from these receptors, other receptors are found in the spleen, such as corticotropin-releasing factor receptors, Fc receptors, atrial natriuretic peptide receptors, the interferon receptor, substance P receptors and tachykinin receptors.

Receptors found in the kidneys include angiotensin, vasopressin, neuropeptide Y, bradykinin, vasoactive intestinal peptide, endothelin, prostaglandin E2, mineralocorticoid, α-adrenergic, dopamine-1, serotonin, β-1 integrin or thiazide diuretic receptors. The mechanism for selective excretion includes binding of the conjugate of the bioactive substance, capturing agent and ligand to a specific kidney receptor, intracellular degradation or modification followed by release to the tubular cells with subsequent secretion.

The lungs have the largest blood flow of all organs and would therefore be well suited for excretion of evaporatable substances. Reticuloendothelial cells are also present in the lungs. Examples of lung receptors which may be suitable for clearance of bioactive susbtances in this fashion are histamine, endothelin, β-adrenergic, interleukin-1, interferon-γ, lung vasoactive intestinal peptide, muscarinic, adenosine, substance P, benzodiazepine, atriopeptin III or leukotriene receptors.

Examples of bone marrow receptors which might be useful for the present purpose are the Fc gamma RI, RII and RIII, interleukin 1 and 2, erythropoietin, transferrin, leukotriene, benzodiazepine or somatostatin receptors.

Due to the extremely large surface area of the skin, it may be a particularly appropriate target for depositing toxic components which cannot be excreted through any other organ without causing damage. Deposited toxic components may either be gradually re-routed to the bloodstream and excreted through another organ (typically the liver) in doses which are small enough not to cause any damage to that organ. The sweat glands may represent a direct route of excretion via appropriate receptors located in the glands (e.g. a vasoactive intestinal polypeptide-like receptor, cf. W. Kummer et al., Neurosci. Lett. 110, 1990, pp. 239-243). Examples of receptors on keratinocytes and skin fibroblasts which might be suitable for the present purpose are low-density lipoprotein, transferrin, interleukin-1 and -2, histamin H1 and H2, glycocorticoid, vitamin D, retinoic acid or retinoic acid gamma receptors or integrin receptors for fibronectin, collagen or laminin.

Intrauterine treatment of foetal disorders is believed to be feasible by the clearance method of the invention by utilizing one of the large number of receptors in the placenta. Examples of such receptors are Fc-γ, LDL, hemopexin, transferrin, α₂-macroglobulin, ferritin, insulin, atrial natriuretic peptide, progesterone, estrogen, glucocorticoid, calcitonin, granulocyte colony stimulating factor 1, adenosine, β-adrenergic, endothelin, interferon-γ, epidermal growth factor, insulin-like growth factor, opioid or thromboxane receptors.

Receptors of the above categories may also be used to modify ligands or ligand analogues, or they may form part of the capturing agent, as indicated above.

Consequently, suitable ligands or ligand analogues may be selected from the group consisting of a growth factor, a peptide or polypeptide hormone, a cytokine, a detoxified toxin, a vitamin, a steroid hormone, or a substance acting on the central nervous system, e.g. a substance capable of binding to any one of the receptors listed above. Other possible ligands may be hyaluronan (cf. B. Smedsrød et al., op. cit.), chondroitin sulphate (cf. B. Smedsrød et al., op. cit.), N-terminal propeptide of collagen type III (cf. B. Smedsrød et al., op. cit.), mannose-terminated glycoconjugates (cf. B. Smedsrød et al., op. cit., or B. Smedsrød et al., Thromb. Haemostas. 59, 1988, pp. 480-484), a glycoside coupled to a bioactive substance capable of binding to the capturing agent (the glycoside binding to the asialoglycoprotein receptor of hepatocytes) or tissue plasminogen activator. In certain special cases, one might contemplate using as the ligand a substance which does not bind to any specific receptor but which is nevertheless bound to a specific organ (e.g. aprotinin which is bound in the kidneys).

The bioactive substance which may be either detected or eliminated by the method of the invention may be a drug (including a narcotic drug), e.g. an alkaloid such as digoxin or morphine, or an antidepressive (including tricyclic antidepressives), acetylsalicylic acid, paracetamol, antiepileptic, β-receptor blocker, neuroleptic, sympatomimetic, a toxin, e.g. an exotoxin or endotoxin (LPS), snake venom, fungal poison (e.g. muscarine), an inorganic poison such as a heavy metal (e.g. lead, cadmium, mercury), an organic poison such as an alkaloid (e.g. nicotine, scopolamine, atropine), a tumour-associated antigen, optionally as present on neoplastic cells, a substance produced by a neoplastic cell, an activated leukocyte, an activated lymphocyte, a plasma cell, surface or circulating substances produced by cells, an antibody, in particular an autoantibody, a thrombus (or a component thereof) or a pathogen (e.g. virus, bacteria, fungi) or a component thereof, such as a surface antigen.

### DESCRIPTION OF SPECIFIC EMBODIMENTS

### I. Detection of vascular thrombi

Vascular thrombi may be rapidly detected by injecting an antibody which is specific for the seven first amino acids of the β-chain of human fibrin. These amino acids are not exposed on fibrinogen which implies that the antibody will react with fibrin, but not with fibrinogen (cf. K.Y. Huy et al., Science 222, 1983, pp. 1129-1132).

More specifically, this fibrin-specific antibody is prepared by the method described in Huy et al., op. cit., and labelled with ¹¹¹In essentially as described by Hnatowich, J. Immun. Meth. 65, 1983, pp. 147-157. By injecting this antibody, any fibrin present in the circulation (e.g. deep venous thrombi or lung emboli) will be labelled with the antibody. After about one hour, a 2-10-fold molar excess of tissue plasminogen activator (tPA) conjugated with the fibrin β-chain heptapeptide mentioned above is then injected. This will result in rapid elimination of the background activity of the labelled antibody in the blood through the liver due to the binding of tPA to hepatic receptors, thereby improving the target-blood ratio of the labelled antibody to a significant degree.

### II. Diagnosis of vascular thrombi

Vascular thrombi may also be detected by a method which is similar to the one described above, but in which a bispecific antibody reactive with the fibrin β-chain heptapeptide as well with tPA is injected and in which background activity is cleared by means of unmodified tPA.

More specifically, monoclonal antibodies reactive with the fibrin β-chain heptapeptide are prepared as described by Huy et al., op. cit. Monoclonal antibodies reactive with human tPA are prepared as described in DK 4082/89. Immunoreactive bispecific antibodies are prepared from the two monoclonal antibodies by the method described in EP 179 872 and labelled with ¹¹¹In essentially as described by Hnatowich, J. Immun. Meth. 65, 1983, pp. 147-157.

By injecting the bispecific antibody, any fibrin present in the circulation (e.g. deep venous thrombi or lung emboli) will be labelled with the antibody. After about one hour, a 2-10-fold molar excess of unmodified tPA (Actilyse®, available from Boehringer Ingelheim) is then injected. This will result in rapid elimination of the background activity of the labelled antibody in the blood through the liver due to the binding of tPA to hepatic receptors, thereby improving the target-blood ratio of the labelled antibody to a significant degree.

### III. Localization of tumours

The localization of tumours by means of radiolabelled antibodies is an established technique (cf., for instance, Chatal, Monoclonal Antibodies in Immunoscintigraphy, CRC, Florida, 1989). It is important to reduce the background activity of the antibody (i.e. establish a high target-blood ratio) in order to obtain a high sensitivity of the diagnosis. Conventionally, this has been done by deferring tumour detection until the level of circulating labelled antibodies has diminished by natural elimination. The clearance of labelled antibodies may be accellerated by injecting a bispecific antibody reactive with a tumour-specific antigen as well as with tPA, followed by injection of tPA after the antibody has been bound to the tumour.

More specifically, monoclonal antibodies reactive with human carcinoembryonic antigen (CEA) is prepared according to standard procedures (cf. J.P. Mach et al., Immunology Today 2, 1981, pp. 239-242). Monoclonal antibodies reactive with human tPA are prepared as described in DK 4082/89. Immunoreactive bispecific antibodies are prepared from the two monoclonal antibodies by the method described in EP 179 872. The bispecific antibodies are labelled with ¹¹¹In essentially as described by Hnatowich, J. Immun. Meth. 65, 1983, pp. 147-157.

The labelled bispecific antibodies are then injected into patients with colorectal cancer and allowed to circulate for about 30 minutes followed by injection of a 2-10-fold molar excess of human tPA (Actilyse®, available from Boehringer Ingelheim). This will result in rapid elimination of the labelled antibody from the circulation to a level at which the tumours are visualized clearly on the scintigrams (a level below about 10-25% of the original level, as determined by experiments with radiolabelled anti-CEA antibodies).

### IV. Treatment of digoxin poisoning

Overdosing of digoxin leading to intoxication represents a serious clinical problem because no efficacious antidote to the drug exists at present. Parenteral administration of antibodies specific for digoxin has previously been suggested in order to reduce the free concentration of the drug (cf., for instance, D.H. Smith and V.P. Butler, J. Clin. Invest. 50, 1971, pp. 1738-1744; W. Clarke and E.A. Ramoska, Am. J. Emerg. Med. 6(5), 1988, pp. 465-470). The clinical efficacy of such treatment varies (S.S. Martiny et al., Crit. Care Med. 16(6), 1988, pp. 629-635). A major drawback of this treatment is that potentially vital digoxin treatment cannot be initiated for quite some time after injection of the antibody.

It may be assumed that more efficient elimination of the circulating antibody-digoxin complex would more rapidly reduce the free concentration of digoxin in the bloodstream. Such elimination may be effected by initially injecting a bispecific antibody reactive with digoxin as well as tPA in the bloodstream of an intoxicated patient. After about 10-30 minutes, tPA is injected in a 2-10-fold molar excess of the antibody. The complexes of digoxin, antibody and tPA (as well as antibody and tPA not bound to digoxin) are then bound to the tPA receptor in the liver (cf. D.C. Rijken et al., Thromb. Res. 57, Suppl. 10, 1990, pp. 63-71) and excreted therefrom.

More specifically, murine monoclonal antibodies reactive with human tPA are produced by the method described in DK 4082/89. Polyclonal antibodies against digoxin are prepared as described by D.H. Smith and V.P. Butler, op. cit. Immunoreactive bispecific antibodies are prepared from the two antibodies by the method described in EP 179 872.

To test the theraputic efficacy of the method of the present invention, four rabbits are treated with theraputic doses of digoxin (NEN, DuPont) for 5 days after which they are intoxicated by administrating an overdose of ³H-labelled digoxin (NEN, DuPont). 6 hours later two of the rabbits are treated with anti-digoxin antibody alone, while the other two rabbits receive an equivalent dose of the bispecific antibody followed by a 2-10-fold molar excess of tPA (e.g. Actilyse®, available from Boehringer Ingelheim) about 30 minutes later. Comparison of the radioactive counts of plasma samples taken from each group of rabbits in the course of the treatment is expected to show a significant decrease of the plasma content of digoxin in the rabbits treated with the bispecific antibody and subsequently injected with tPA.

### V. Elimination of LPS from the circulation

Endotoxin (LPS) plays a significant part in the pathogenesis of gram-negative septicemia (cf. T.B. Casale et al., J. Allergy Clin. Immunol. 85, 1990, pp. 45-51). It has previously been attempted, with varying degrees of success, to neutralize LPS by administering polyclonal or monoclonal anti-LPS antibodies (cf. L.S. Young et al., Rev. Infect. Dis. 11, Suppl. 7, 1989, pp. S1564-S1571; B.J. Appelmelk et al., Microb. Pathogen 5, 1988, pp. 251-257). A major obstacle to this approach is that most anti-LPS antibodies do not protect against LPS toxicity. It is contemplated that elimination of LSP by the liver may be increased by administering a bispecific antibody which is reactive with LPS as well as with tPA, followed by injection of a suitable amount of tPA.

More specifically, monoclonal antibodies reactive with LPS are prepared according to standard procedures (cf. M.N. Nys et al., J. Infect. Dis. 162, 1990, pp. 1089-1095). Monoclonal antibodies reactive with human tPA are prepared as described in DK 4082/89. Immunoreactive bispecific antibodies are prepared from the two monoclonal antibodies by the method described in EP 179 872.

The bispecific antibodies are then injected into septicemic patients and allowed to circulate for about 30 to 60 minutes to bind a significant proportion of the endotoxin present, followed by injection of a 2-10-fold molar excess of human tPA (Actilyse®, available from Boehringer Ingelheim). This will result in a more rapid elimination of the LPS/antibody/tPA complex from the circulation.

### BRIEF DESCRIPTION OF THE DRAWINGS

Fig. 1 is a graph showing the total radioactivity in plasma of In-111 labelled anti-t-PA monoclonal antibody (MoAb) with and without subsequent injection of t-PA.

Fig. 2 is a graph showing the immunoreactive part of In-111 labelled anti-t-PA MoAb with and without subsequent injection of t-PA.

Fig. 3 is a scintigram showing the organ distribution in rabbits of In-111 labelled anti-t-PA MoAb alone and complexed with t-PA.

Fig. 4 is a graph showing the total radioactivity in human plasma of In-111 labelled anti-t-PA MoAb before and after injection of t-PA.

Fig. 5 is a graph showing the change in human organ distribution of In-111 labelled anti-t-PA MoAb following injection of t-Pa.

Fig. 6 shows two scintigrams of the crural region initially showing increased accumulation of In-111 labelled anti-t-PA MoAb in the vascular bed of the right leg (A), and after t-PA injection revealing the focal thrombus area (top of the right leg) (B).

Fig. 7 is a graph showing gelfiltration of a bispecific anti-t-PA/anti-rabbit antibody. All fractions were tested for OD₂₈₀ and reactivity in a t-PA enzyme immunoassay (EIA). All fractions were diluted 1:25000 before testing in the EIA system.

Fig. 8 is a graph showing the concentration, determined by EIA, of a bispecific anti-t-PA/anti-rabbit antibody in rabbit plasma with (indicated by squares) and without (indicated by triangles) injection of t-PA.

Fig. 9 is a graph showing the total amount, determined by EIA, of biotinylated bispecific anti-t-PA/anti-rabbit antibody in rabbit plasma with (indicated by squares) and without (indicated by triangles) injection of t-PA.

The present invention is illustrated in further detail in the following examples which are not in any way intended to limit the scope of the invention as claimed.

### Example 1

### Monofunctional capturing agent

### (a) Production of a monoclonal antibody against t-PA

### Purification of t-PA antigen

The t-PA used for the immunization of mice was purified as disclosed in Rijken, D.C. and Collen, D., J. Biol. Chem. 256, 1981, pp. 7035-41. The purified material contained >95% pure t-PA as determined by silver-stained SDS-polyacrylamide gel electrophoresis.

### Immunization of AKR mice with t-PA

Purified t-PA obtained as described above was dialysed against 0.15 M NaCl containing 0.01% (v/v) Tween 80, and the concentration was adjusted to 10 »g/ml.

AKR mice were immunized 3 times at bi-weekly intervals. For the first two immunizations, each mouse was injected subcutaneously with 50 »l t-PA emulsified with 50 »l of Freunds's incomplete adjuvant (corresponding to 5 »g of t-PA/mouse). The last immunization was identical to the first two, but was given intraperitoneally rather than subcutaneously. Two months later a mouse received an intravenous booster injection of 90 »l t-PA without any Freund's adjuvant.

### Cell fusion and culture of cells

Three days after the intravenous t-PA booster, the spleen was removed, and a spleen cell suspension was prepared by carefully dissecting and disrupting the spleen. The resulting spleen cells were used for cell fusions as described in Petersen, L.C. et al., Thrombosis and Haemostasis 57 (2), 1987, pp. 205-211. In brief, spleen cells were fused with X63-Ag8-6.5.3 myeloma cells in the presence of a polyethylene glycol solution. After fusion the cells were seeded in 10 96-well microtiter cell plates. Hybridoma supernatants were screened after two weeks growth by enzyme-linked immunosorbent assay using t-PA as the antigen. A positive clone was recloned several times by the technique of limiting dilution to ensure monoclonality. The cell line was then grown on a large scale in "cell factories" from NUNC in a medium consisting of RPMI 1640 (Gibco, UK) with 2% BMS (Gibco, UK).

### Purification of the monoclonal antibody

The hybridoma supernatants were concentrated. pH was adjusted to 8.3, after which the monoclonal antibody was purified at 4°C by adsorption to on a Protein A gel. Elution was performed with a citrate buffer pH 4.5.

### Production of F(ab)₂ fragments

The purified monoclonal antibody (subclass IgG₁) was digested with pepsin at a enzyme/substrate ratio of 1:100 at pH 4.30 for 16 h at 37°C. The reaction was stopped by adjusting the pH of the reaction mixture to pH 8.5 with solid Tris. The F(ab)₂ was separated from undigested IgG and low molecular weight products on a Ultrogel AcA 44 column. Purification of the antibody, pepsin digestion and purification of F(ab)₂ was monitored on silver-stained SDS-gels.

### Labelling of F(ab)₂ with indium

The purified F(ab)₂ was labelled with indium essentially according to the method of Hnatowich (J.Immun.Methods. **65**, 147-157 (1983).Immunoreactivity of conjugates was confirmed by ELISA and with immunosorbent technique using t-PA as antigen.

The immunochemical purity of the labelled antibody was determined as the percentage of antibody radioactivity capable of binding to a t-PA coupled Sepharose (Pharmacia AB, Sweden) column. The immunochemical purity of the conjugate was less than the radiochemical purity since some of the labelled antibody was immunological by non-reactive. The immunochemical purity of the antibody was 93%.

### (b) Biological background subtraction - animal study

### t-PA

The t-PA used in the experiments was Actilyse obtained from Boehringer Ingelheim.

### Experimental design

Two rabbits were injected intravenously with the antibody in dose/kg and specific radioactivity comparable to the human studies mentioned in Example 2.

Rabbit 1 received antibody at t = 0 minutes. No t-PA was subsequently administered. Rabbit 2 received antibody at t = 0 minutes. At t = 120 minutes rabbit 2 received an intravenous injection of t-PA administered over a period of 1 minute. The injected amount of t-PA was in 3 x molar concentration of the administered antibody.

### Plasma activity

Blood samples were taken at t = 0, 10, 20, 40, 60 min and 2, 4, 6, 12, and 24 hours after the antibody injection. In rabbit 2 additional samples were taken 5, 10, 20, 40, and 60 min after the injection of t-PA.

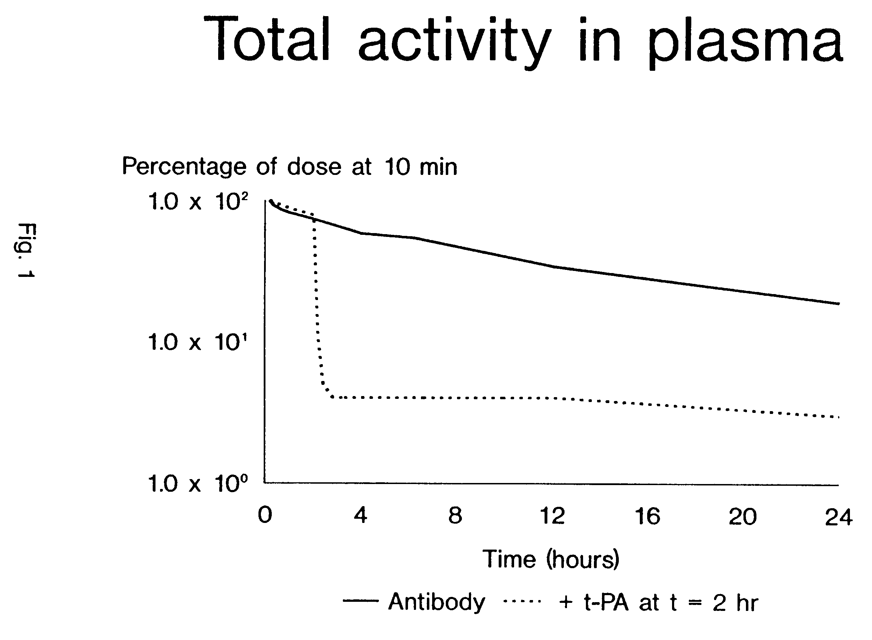

For each sample, whole plasma activity was counted, and by regression analysis (Freelance Plus ver 3.01) these data were adjusted to an exponential curve and the overall biological half-life was calculated. All curves presented were corrected for physical decay. The results are shown in Fig. 1.

T½ of the antibody was 10.3 h.

When t-PA was added at t = 2 hours a dramatic decrease in plasma activity was observed. Within 10 min a 92% reduction was observed.

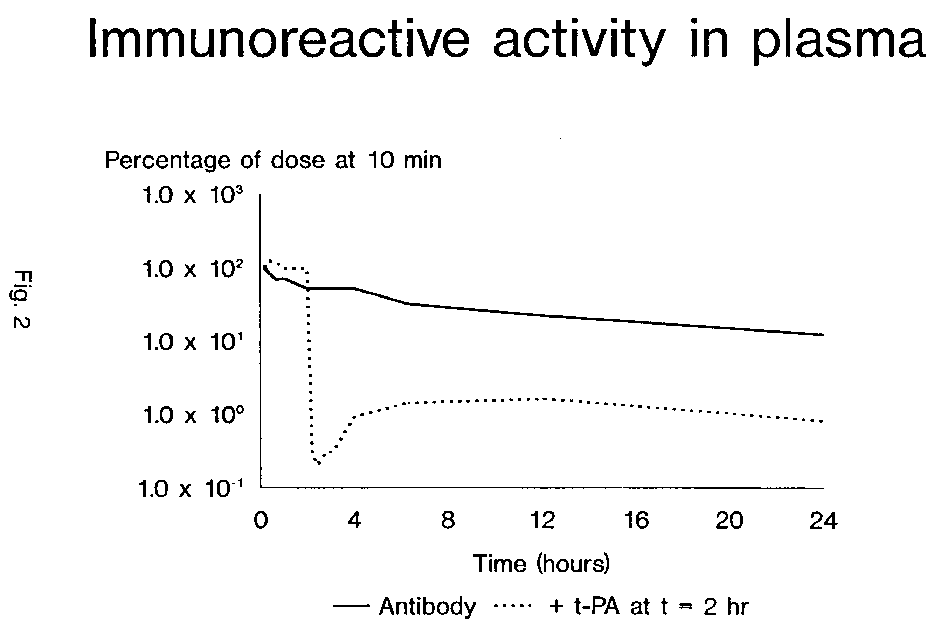

The biological half-life of the radiolabelled immunoreactive antibody in plasma was determined as the half-life of t-PA binding activity. The results are shown in Fig. 2.

Within 10 minutes after the injection of t-PA at t = 2 hours more than 99% of the immunoreactive antibody activity was eliminated from the plasma. The half-life of this elimination is 1 minute. A slight increase in activity - up to 1.6% - is seen in the hours after the initial elimination. This is undoubtedly caused by redistribution of the extravascular antibody pool.

### Whole body activity

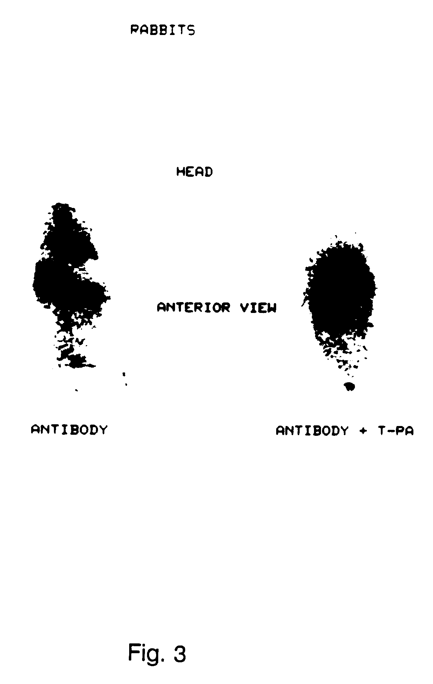

After 24 hours the rabbits were sacrificed. Whole body scintigrams of the rabbits were obtained. The results are shown in Fig. 3. Antibody not complexed with t-PA was mainly located in the kidneys, whereas antibody complexed with t-PA was almost exclusively found in the liver.

It appears from these experiments that t-PA/anti-t-PA MoAb complexes have a substantially shorter half-life in the organism than anti-t-PA alone. Furthermore, the experiments show that is is possible to exploit the rapid t-PA/anti-t-PA MoAb clearance in vivo. The results of the study suggest that the hepatic t-PA receptor is responsible for this increase in antibody clearance.

The decline in total radioactivity is an essential parameter for imaging, in that the total plasma activity will decide the target/blood ratio. The reduction in t-PA binding radioactivity is a measure for the in vivo efficiency of the receptor elimination pathway. The decrease in total radioactivity will be less than the reduction in t-PA binding radioactivity unless the conjugate is 100% immunochemically active.

### Example 2

### Biological background substraction - human study

A 77 year-old woman was admitted to hospital because of pronounced oedema of the right leg. An acute contrast phlebography exposed signs of a thrombosis in one of the lateral crural veins of the right leg. Scintigraphy was performed using a radiolabelled monoclonal antibody prepared as described in example 1. The radiochemical purity was only 67%.

One hour after injection of the radiolabelled antibody 5 mg of t-PA (Actilyse Boehringer Ingelheim) was injected.

### Plasma activity

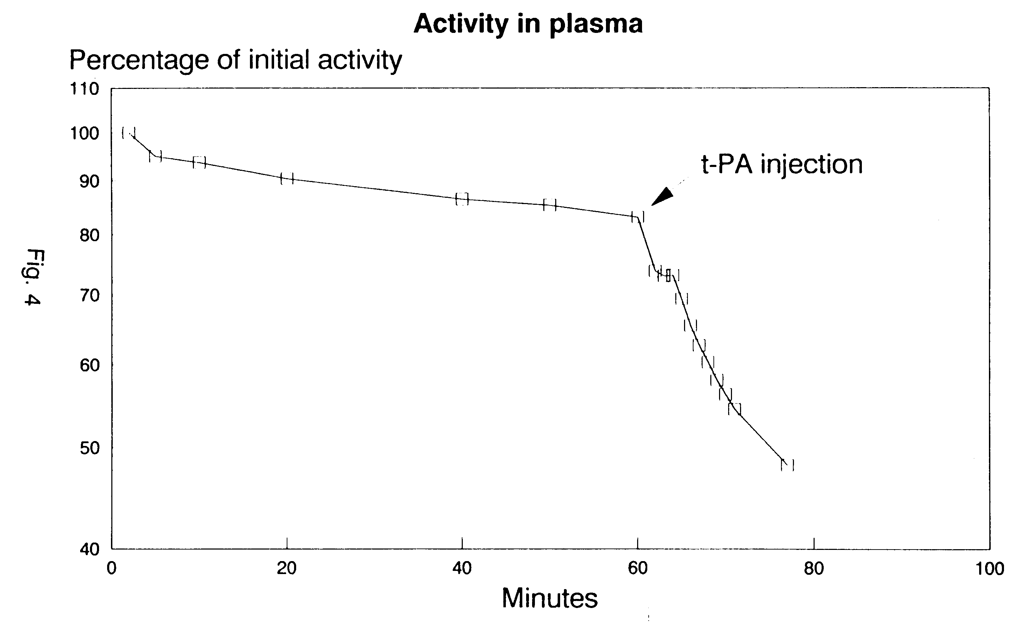

Blood samples were taken at regular intervals after the t-PA injection. For each sample, whole plasma radioactivity was counted in a well gamma-counter and plotted against time (Fig. 4). Exponential regression analysis was performed on Freelance Plus ver 3.01). T½ of the initial phase was 4.8 h. After t-PA injection a substantial decrease in plasma activity was observed. Within 17 min a 50% reduction was observed.

### Scintigraphy

### Biodistribution:

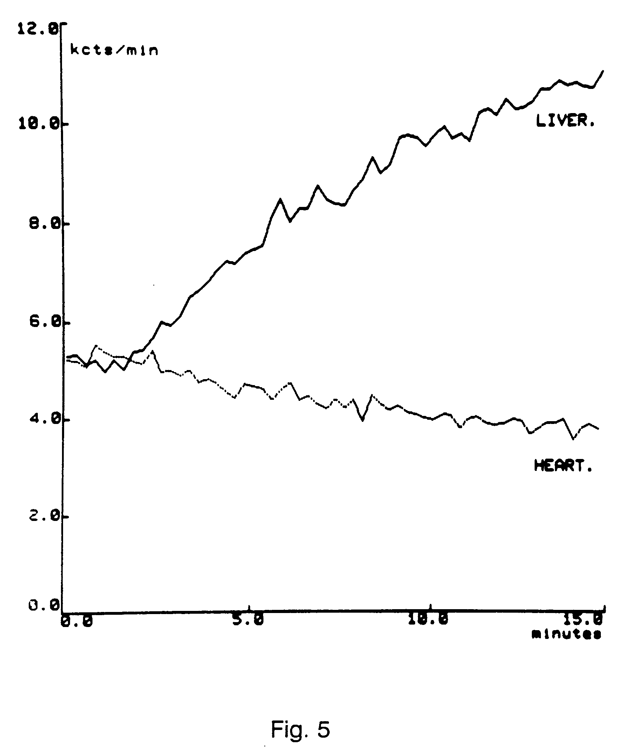

During t-PA injection a dynamic acquisition of the liver and heart region was performed on the gamma-camera with 60 frames lasting 15 sec each. Activity of the blood (heart region) and the liver were calculated and plotted against time. The shift in distribution from circulating blood activity to bound liver activity following injection is clearly demonstrated on Fig. 5.

### Thrombus detection:

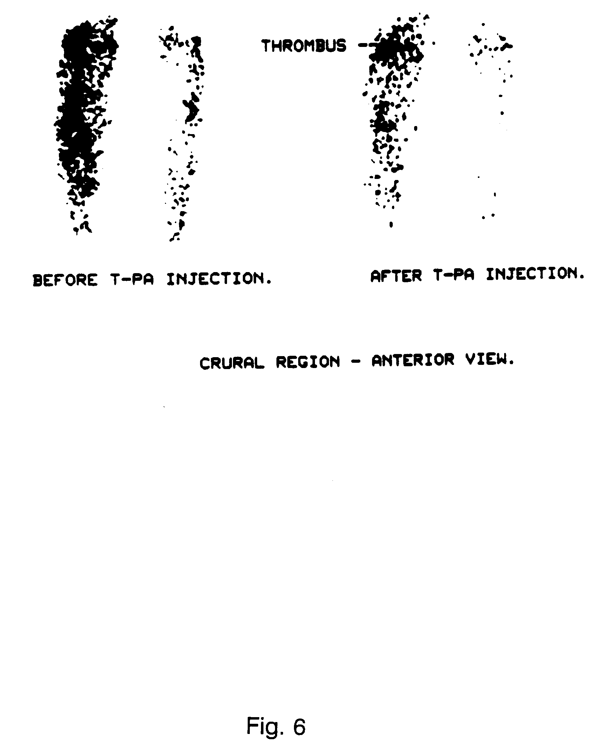

Initially, signs of stasis in the lower leg were revealed. However, the precise location of the thrombus could not be detected. After the t-PA injection, the activity in the dilated vascular bed diminished, and the thrombus area was detected (Fig. 6).

The biological background subtraction appears to improve the imaging technique, making it possible to detect thrombosis within a few hours.

### Example 3

### Bifunctional capturing agent

### (a) Production of a monoclonal antibody against t-PA

### Purification of t-PA antigen

The t-PA used for the immunization of mice was purified as disclosed in Rijken, D.C. and Collen, D., J. Biol. Chem. 256, 1981, pp. 7035-41. The purified material contained >95% pure t-PA as determined by silver-stained SDS-polyacrylamide gel electrophoresis.

### Immunization of RBF/Dn mice with t-PA

Purified t-PA obtained as described above was dialysed against 0.15 M NaCl containing 0.01% (v/v) Tween 80, and the concentration was adjusted to 10 »g/ml.

### Origin of parent myeloma cell line:

The myeloma cells used for production of the monoclonal antibody 3 F1 were the FOX-NY myeloma line deficient in the selectable enzyme marker locus adenosine phosphoribosyl transferase (ARPT⁻) and hypoxanthine phosphoribosyl transferase (HRPT⁻). This myeloma cell line is "non-producing" cell line, ie. it does not secrete or synthesize any immunglobulin chains.

### Origin of animal strain

RBF/Dn-strain mice (obtained from the Jackson Laboratory, Bar Harbor, Maine, USA) containing the RB (8.12)5Bnr Robertsonian translocation chromosome were used for production of monoclonal antibodies.

### Immunization and fusion

RBF/Dn-strain mice were immunized three times at bi-weekly intervals with purified human t-PA. Antigen was emulsified 1:1 in Freund's incomplete adjuvant prior to immunization of the mice. Each mouse received 10 »g antigen with each immunization in a total volumen of 200 »l. One month after the last immunization a mouse was boosted intravenously with 10 »g human t-PA. After another three days the mouse was sacrificed and the spleen removed for fusion with myeloma cells.

Spleen cells from one mouse (10.2 x 10⁷ cells) were fused with 2.5 x 10⁷ cells of the FOX-NY cell line. The fused cells were seeded on BALB/strain mouse macrophage feeder cells in a total of 10 96-well microtiter plates in a medium consisting of RPMI-1640 with 15 % v/v fetal calf serum (Gibco of Flow) supplemented with adenine (7.5 x 10⁻⁵ M), hypoxanthine (8 x 10⁻⁴ M, aminopterin (8 x 10⁻⁷ M) and thymidine (1.6 x 10⁻⁵ M) (AHAT). Consequently, the exposure of cell fusion mixture to a medium requiring ARPT activity (ARPT⁺ selection) eliminates both unfused ARPT⁻ myelomas and ARPT⁻ hybridomas. This results in increased stability of immunoglobulin producing hybridomas (R.T.Taggart and I.M.Samloff: Science 219 (1983) pp. 1228 - 1230).

Hybridoma supernatants were screened after two weeks' growth by enzyme linked immunosorbent assay using t-PA as the antigen. A positive clone was recloned several times by the technique of limiting dilution to ensure monoclonality. The cell line was then grown on a large scale in "cell factories" from NUNC in a medium consisting of RPMI 1640 (Gibco, UK) with 2% BMS (Gibco, UK).

### Purification of the monoclonal antibody

The hybridoma supernatants were concentrated. The pH was adjusted to 8.3, after which the monoclonal antibody was purified at 4°C by adsorption to on a Protein A gel. Elution was performed with a citrate buffer pH 4.5.

### Production of F(ab)₂ fragments

The purified monoclonal antibody (subclass IgG₁) was digested with pepsin at a enzyme/substrate ratio of 1:100 at a pH of 4.10 for 16 h at 37°C. The reaction was stopped by adjusting the pH of the reaction mixture to pH 8.5 with solid Tris. The F(ab)₂ was separated from undigested IgG on a Protein A column and further purification was performed on a Ultrogel AcA 44 column. Purification of the antibody, pepsin digestion and purification of F(ab)₂ was monitored on Coomassie-stained SDS-gels.

### Purification of rabbit immunoglobulin

Rabbit serum was adjusted to a pH of 7.5, after which the rabbit immunoglobulin was purified at 4°C by adsorption to on a Protein A gel. Elution was performed with a citrate buffer pH 3.5.

### Production af rabbit F(ab)₂ fragments

The purified rabbit immunoglobulin was digested with pepsin at a enzyme/substrate ratio of 1:100 at pH 4.50 for 16 h at 37°C. The reaction was stopped by adjusting the pH of the reaction mixture to pH 8.5 with solid Tris. The F(ab)₂ was separated from undigested IgG on a Protein A column and further purification was performed on a Ultrogel AcA 44 column. Purification of the antibody, pepsin digestion and purification of F(ab)₂ was monitored on Coomassie-stained SDS-gels.

### Biotinylation of rabbit immunoglobulin

The purified rabbit F(ab)₂ was biotinylated with Biotinyl-ε-aminocaproic acid N-hydroxysuccinamidester (biotin-x-NHS)-(Calbiochem 203188). Immediately prior to use, biotin-x-NHS is dissolved in DMSO. The biotinylation was performed in a PBS buffer pH 7.2 at 20°C with a molar ratio of biotin-x-NHS/F(ab)₂ of 100. The biotinylation process was stopped after 2 hours by addition of 10 % v/v of imidazol buffer, 0.5 M pH 7.3.

### Preparation of a bifunctional F(ab)₂

Preparation of the bifunctional antibody was performed essentially according to Glennie, M.J. et al. J.Immunol. vol 139, page 2367-2375 1987.

### Reduction of biotinylated rabbit F(ab)₂

1 ml purified biotinylated rabbitF(ab)₂ at a concentration of 10 mg/ml was reduced with 10 mM DTE flushed with nitrogen and stored for 60 minutes at 20°C followed by 30 minutes at 4°C. Separation af Fab from DTE was performed on a 1.5x15 cm Sephadex G-25 column equilibrated with 50 mM acetate buffer ph 5.3 with 2 mM EDTA. The eluted Fab was pooled and stored on ice before use.

### Reduction and maleimide coupling of murine F(ab)₂

1 ml purified monoclonal anti t-PA F(ab)₂ at a concentration of 10 mg/ml was reduced with 10 mM DTE, flushed with nitrogen and stored for 60 minutes at 20°C followed by 30 minutes at 4°C. Separation af Fab from DTE was performed on a 1.5x15 cm Sephadex G-25 column equilibrated with 50 mM acetate buffer pH 5.3 containing 2 mM EDTA. The eluted Fab was pooled. To 3.2 ml Fab at a concentration of 1.55 mg/ml was added 1.6 ml 12 mM mM o-phenylene dimaleimide (o-PDM, SIGMA) dissolved in chilled dimethylformamide. After 30 minutes the maleidated Fabₘₐₗ was separated from the other solutes in the reaction mixture by passage through a 2.5x 15 cm Sephadex G-25 column equilibrated with 50 mM sodium acetate pH 5.3 containing 2 mM EDTA.

### Coupling of murine Fabₘₐₗ with biotinylated rabbit Fab

x₁ ml Fabₘₐₗ at a concentration of 0.41 mg/ml (82 nmol) was mixed with 3.3 ml biotinylated rabbit Fab at a concentration of 1.62 mg/ml (107 nmol). The reaction mixture was concentrated to 2 mg/ml by ultrafiltration under nitrogen using a Amicon YM-10 membrane in a chilled Amicon chamber (Amicon Ltd).

### Gelfiltration of bispecific antibody

The reaction mixture was gelfiltrated an a 1.5x100 cm Ultrogel AcA44 column equilibrated with 0.2 M phosphate buffer pH 6.0 containing 5 mM EDTA. The flow rate was 8 ml/h and the fractions were of 1 ml.

### EIA test of gelfiltrated bispecific antibody

All fractions were assayed by EIA. 96-well plates were coated with human t-PA at a concentration of 1 »g/ml for 16 hours at room temperature. After blocking the plates, all fractions were assayed in this EIA. Fractions were tested in dilutions of 1:500 and 1:25000 in a PBS buffer containing 0.02% Tween 20 supplemented with 1 % BSA. Plates were washed after incubation for 1 hour. The plates were then incubated with streptavidin-peroxidase (Kirkegård and Perry Laboratories, Inc) in a dilution of 1:20000 in a PBS buffer supplemented with 0.5 M NaCl. After a final washing, the wells were incubated with 100 »l substrate buffer (citrate perborate buffer, pH 5.5, containing ortho-phenylenediamine, 0.8 mg/ml) for 5 minutes, after which the reaction was stopped by addition of 100 »l 2 M sulfuric acid to each well. Plates were read at 492 nm.

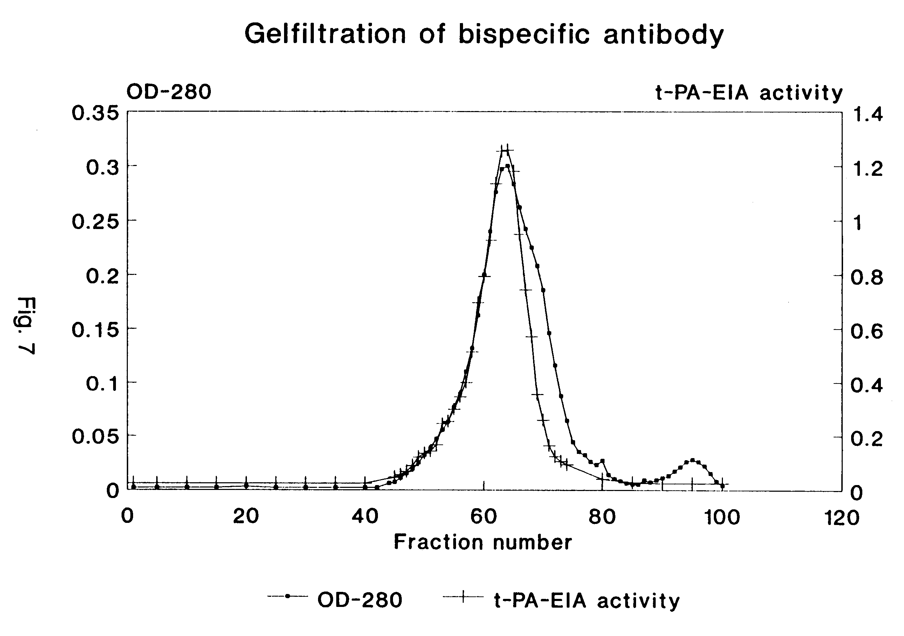

Results are shown in Fig. 7. In this figure, absorbance in the EIA of all fractions diluted 1/x is depicted.

Fractions containing bispecific antibodies as estimated by the EIA were pooled. The volume was 6 ml and the antibody concentration (determined as A₂₈₀) was 198 »g/ml. This pool af bispecific antibody was used for elimination experiments.

### (b) Biological background subtraction

### t-PA

The t-PA used in the experiments was Actilyse obtained from Boehringer Ingelheim.

### Experimental design

Four rabbits were injected intravenously with the bispecific antibody in a dose of 50 »g/rabbit.

Rabbit 1 and 2 received antibody at t = 0 minutes. No t-PA was subsequently administered. Rabbit 3 and 4 received antibody at t = 0 minutes. At t = 60 minutes rabbit 3 and 4 received an intravenous injection of t-PA administered over a period of 1 minute. The injected amount of t-PA was in 12 x molar concentration of the administered antibody.

### Plasma activity

Blood samples were taken at t = 0, 10, 20, 40, 60 min and 2, 4, 6 after the antibody injection. In rabbit 3 and 4 additional samples were taken 5, 10, 20, 40, and 60 min after the injection of t-PA.

The concentration of bispecific antibody was determined in the above mentioned EIA using a calibration curve (200 ng/ml to 0.78 ng/ml) of bispecific antibody in rabbit plasma. All samples were tested undiluted and diluted 1:10 in normal rabbit plasma.

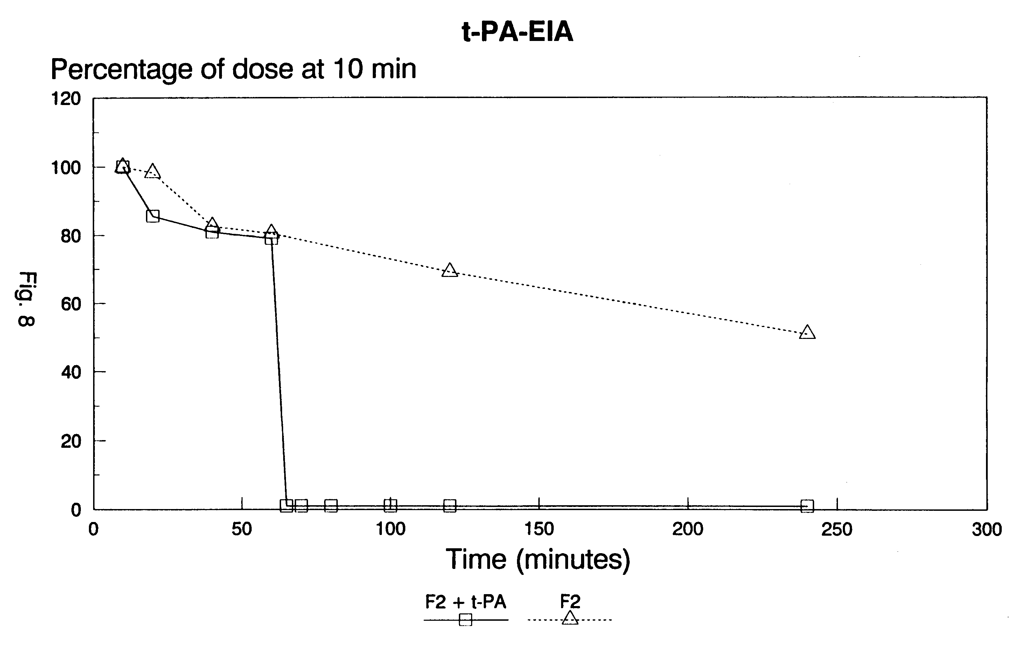

The results are shown in Fig. 8 in which each curve represents the mean value of two rabbit plasma samples. The half-life of the bispecific antibody in the control rabbits that did not receive any t-PA was approximately 4.5 hours. When t-PA was added at t = 1 hours a dramatic decrease in the serum concentration of the bispecific antibody was observed. Within 10 min substantially all of the bispecific antibody had been cleared from the bloodstream as evaluated by the EIA results.

The results were verified in an EIA which measured the total amount of biotinylated antibody using a microtiter plate in which the wells were coated with streptavidin. The concentration of bispecific antibody in rabbit plasma was determined in this EIA using a calibration curve (200 ng/ml to 0.78 ng/ml) of bispecific antibody in rabbit plasma. The response of this calibration curve was unaffected by the addition of high concentrations of t-PA. Samples were incubated undiluted and diluted 1:10 in normal rabbit plasma. CAlibrators and samples were incubated for 1 hour. The plates were then incubated with streptavidin-peroxidase (Kirkegård and Perry Laboratories, Inc) in a dilution of 1:20000 in a PBS buffer supplemented with 0.5 M NaCl. After a final washing, the wells were incubated with 100 »l substrate buffer (citrate perborate buffer pH 5.5 supplemented with ortho-phenylenediamine 0.8 mg/ml) for 5 minutes, after which the reaction was stopped by addition of 100 »l 2 M sulfuric acid to each well. Plates were read at 492 nm.

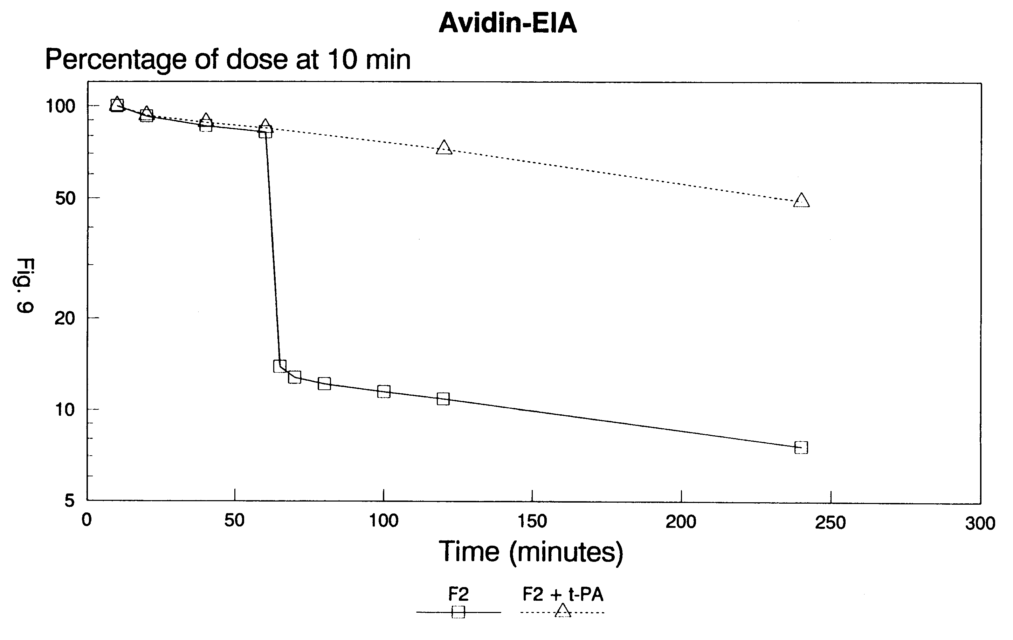

The results of are shown in Fig. 9 in which each curve represents the mean value of two rabbit plasma samples. All antibody concentrations are given as percentages, antibody concentrations measured after 10 minutes being set at 100%. The half-life of the bispecific antibody in control rabbits that dis not receive any t-PA was approximately 4 hours. Within 10 minutes after the administration of t-PA, a decrease in the concentration of biotinylated bispecific antibody of approximately 80% was observed.

The results of this experiment show that the elimination of bispecific antibody as shown in Fig. 9 is not simply caused by a reaction between the bispecific antibody and t-PA making the compleks unable to bind to the solid phase t-PA in the EIA plate.

It appears from these experiments that t-PA/anti-t-PA bispecific antibodies have a substantially shorter half-life in the organism than bispecific antibodies alone. Furthermore, the experiments show that is is possible to exploit the rapid t-PA/anti-t-PA MoAb clearance in vivo.

## Claims

1. A pharmaceutical or diagnostic composition comprising, in separate containers,
(a) a capturing agent capable of binding to a bioactive substance so as to make it possible to locate, neutralize and/or remove the bioactive substance on administration of the capturing agent; and
(b) a ligand or ligand analogue capable of binding to a cellular receptor found in an organ through which waste products of the body are usually eliminated,
the capturing agent being provided with means to bind the ligand or ligand analogue, or the ligand or ligand analogue being provided with means to bind the capturing agent, or both the capturing agent and the ligand or ligand analogue being provided with complementary binding means.

2. A composition according to claim 1, wherein the capturing agent is an agent to be cleared rapidly from the circulation.

3. A composition according to claim 2, wherein the ligand or ligand analogue is modified so as to comprise an antibody (including an anti-idiotype antibody) capable of binding to the capturing agent, a cellular receptor (other than the specific ligand-binding receptor) or a fragment thereof including the binding site for the capturing agent, or wherein the ligand or ligand analogue is modified with a bioactive substance or a derivative or fragment thereof capable of binding the capturing agent.

4. A composition according to claim 2, wherein the bioactive substance and the ligand are substantially identical.

5. A composition according to claim 1, wherein the bioactive substance is a substance to be cleared rapidly from the circulation.

6. A composition according to claim 5, wherein the bioactive substance is different from the ligand or ligand analogue which is modified so as to comprise an antibody (including an anti-idiotype antibody) capable of binding to the capturing agent, a cellular receptor (other than the specific ligand-binding receptor) or a fragment thereof including the binding site for the capturing agent, or wherein the ligand or ligand analogue is modified with a bioactive substance or a derivative or fragment thereof capable of binding the capturing agent.

7. A composition according to claim 5, wherein the bioactive substance is different from the ligand or ligand analogue.

8. A composition according to claim 7, wherein the ligand or ligand analogue is modified so as to comprise an antibody (including an anti-idiotype antibody) capable of binding to the capturing agent, a cellular receptor (other than the specific ligand-binding receptor) or a fragment thereof including the binding site for the capturing agent, or wherein the ligand or ligand analogue is modified with a bioactive substance or a derivative or fragment thereof capable of binding the capturing agent.

9. A composition according to claim 2 or 4, wherein the capturing agent comprises an antibody or a cellular receptor (other than the specific ligand-binding receptor) or a fragment thereof including the binding site for the bioactive substance.

10. A composition according to claim 7, wherein the capturing agent comprises
(a) a bispecific antibody,
(b) a conjugate of an antibody and a cellular receptor (other than the specific ligand-binding receptor) or a fragment thereof comprising the binding site for the bioactive substance, a single-stranded oligonucleotide, an intercellular adhesion molecule, or a complexing agent (e.g. biotin, avidin, lectin or a drug), or
(c) a conjugate of a cellular receptor (other than the specific ligand-binding receptor) or a fragment thereof comprising the binding site for the bioactive substance and a single-stranded oligonucleotide, an intercellular adhesion molecule, or a complexing agent (e.g. biotin, avidin, lectin or a drug).

11. A composition according to claim 10, wherein the ligand or ligand analogue is modified so as to comprise an antibody, a cellular receptor (other than the ligand-binding receptor) or a fragment thereof comprising a binding site for the capturing agent, a single-stranded oligonucleotide, an intercellular adhesion molecule or a complexing agent, each of which is capable of forming a conjugate with the part of the capturing agent which does not bind the bioactive substance.

12. A composition according to any of claims 1-11, wherein the capturing agent is provided with a label.

13. A composition according to claim 12, wherein the label is selected from the group consisting of radioactive isotopes, magnetite particles, and paramagnetic atoms.

14. A composition according to any of claims 1-13, wherein the cellular receptor is a liver, spleen, bone marrow, lung, kidney, skin or placental receptor.

15. A composition according to any of claims 1-14, wherein the bioactive substance is a drug (including a narcotic drug), e.g. an alkaloid such as digoxin or morphine, or an antidepressive (including tricyclic antidepressives), acetylsalicylic acid, paracetamol, antiepileptic, β-receptor blocker, neuroleptic, sympatomimetic, a toxin, e.g. an exotoxin or endotoxin (LPS), snake venom, fungal poison (e.g. muscarine), an inorganic poison such as a heavy metal (e.g. lead, cadmium, mercury), an organic poison such as an alkaloid (e.g. nicotine, scopolamine, atropine), a tumour-associated antigen, optionally as present on neoplastic cells, a substance produced by a neoplastic cell, an activated leukocyte, an activated lymphocyte, a plasma cell, surface or circulating substances produced by cells, an antibody, in particular an autoantibody, a thrombus (or a component thereof) or a pathogen (e.g. virus, bacteria, fungi) or a component thereof, such as a surface antigen.

16. A composition according to any of claims 1-15, wherein the ligand or ligand analogue is a growth factor, a peptide or polypeptide hormone, a cytokine, a detoxified toxin, a vitamin, a steroid hormone, a substance acting on the central nervous system, hyaluronan, chondroitin, sulfate, N-terminal peptide of collagen type III, mannose-terminated glycoconjugates, a glycoside (coupled to a bioactive substance) or tissue plasminogen activator.

17. Use of a capturing agent and a ligand or ligand analogue for the manufacture of a pharmaceutical composition providing the rapid clearance of a bioactive substance from the circulation and comprising
(a) a capturing agent capable of binding to a bioactive substance so as to make it possible to locate, neutralize and/or remove the bioactive substance on administration of the capturing agent; and
(b) a ligand or ligand analogue capable of binding to a cellular receptor found in an organ through which waste products of the body are usually eliminated,
the capturing agent being provided with means to bind the ligand or ligand analogue, or the ligand or ligand analogue being provided with means to bind the capturing agent, or both the capturing agent and the ligand or ligand analogue being provided with complementary binding means,
the composition being adapted for separate and sequential administration of the capturing agent and the ligand or ligand analogue.

18. Use of a capturing agent and a ligand or ligand analogue for the manufacture of a diagnostic composition providing the rapid clearance of the capturing agent from the circulation and hence a rapid detection of the pathological condition in question, the composition comprising
(a) a capturing agent capable of binding to a bioactive substance so as to make it possible to locate, neutralize and/or remove the bioactive substance on administration of the capturing agent; and
(b) a ligand or ligand analogue capable of binding to a cellular receptor found in an organ through which waste products of the body are usually eliminated,
the capturing agent being provided with means to bind the ligand or ligand analogue, or the ligand or ligand analogue being provided with means to bind the capturing agent, or both the capturing agent and the ligand or ligand analogue being provided with complementary binding means,
the composition being adapted for separate and sequential administration of the capturing agent and the ligand or ligand analogue.

## Patentansprüche

1. Eine pharmazeutische oder diagnostische Zusammensetzung, die, in getrennten Behältern, umfaßt
(a) ein Abfangmittel, das in der Lage ist, sich an eine bioaktive Substanz zu binden, um es möglich zu machen, die bioaktive Substanz bei Verabreichung des Abfangmittels zu lokalisieren, zu neutralisieren und/oder zu entfernen; und
(b) einen Liganden oder ein Ligandenanalog, der (das) in der Lage ist, sich an einen zellulären Rezeptor zu binden, der in einem Organ anzutreffen ist, durch das Abfallprodukte des Körpers üblicherweise ausgeschieden werden,
wobei das Abfangmittel mit Mitteln zur Bindung des Liganden oder des Ligandenanalogs versehen ist oder der Ligand oder das Ligandenanalog mit Mitteln zur Bindung des Abfangmittels versehen ist oder sowohl das Abfangmittel als auch der Ligand oder das Ligandenanalog mit komplementären Bindungsmitteln versehen sind.

2. Eine Zusammensetzung nach Anspruch 1, wobei das Abfangmittel ein schnell aus dem Kreislauf auszuscheidendes Mittel ist.

3. Eine Zusammensetzung nach Anspruch 2, wobei der Ligand oder das Ligandenanalog modifiziert ist, um einen Antikörper (einschließlich eines anti-Idiotyp-Antikörpers), der in der Lage ist, sich an das Abfangmittel zu binden, einen zellulären Rezeptor (verschieden von dem spezifischen Ligandenbindungsrezeptor) oder ein Fragment davon, einschließlich der Bindungsstelle für das Abfangmittel, zu umfassen, oder wobei der Ligand oder das Ligandenanalog modifiziert ist mit einer bioaktiven Substanz oder einem Derivat oder Fragment davon, die (das) in der Lage ist, sich an das Abfangmittel zu binden.

4. Eine Zusammensetzung nach Anspruch 2, wobei die bioaktive Substanz und der Ligand im wesentlichen identisch sind.

5. Eine Zusammensetzung nach Anspruch 1, wobei die bioaktive Substanz eine schnell aus dem Kreislauf auszuscheidende Substanz ist.

6. Eine Zusammensetzung nach Anspruch 5, wobei die bioaktive Substanz verschieden ist vom Liganden oder Ligandenanalog, der (das) modifiziert ist, um einen Antikörper (einschließlich eines anti-Idiotyp-Antikörpers), der in der Lage ist, sich an das Abfangmittel zu binden, einen zellulären Rezeptor (verschieden vom spezifischen Ligandenbindungsrezeptor) oder ein Fragment davon, einschließlich der Bindungsstelle für das Abfangmittel, zu umfassen, oder wobei der Ligand oder das Ligandenanalog modifiziert ist mit einer bioaktiven Substanz oder einem Derivat oder Fragment davon, die (das) in der Lage ist, sich an das Abfangmittel zu binden.

7. Eine Zusammensetung nach Anspruch 5, wobei die bioaktive Substanz verschieden vom Liganden oder Ligandenanalog ist.

8. Eine Zusammensetzung nach Anspruch 7, wobei der Ligand oder das Ligandenanalog modifiziert ist, um einen Antikörper (einschließlich eines anti-Idiotyp-Antikörpers), der in der Lage ist, sich an das Abfangmittel zu binden, einen zellulären Rezeptor (verschieden vom spezifischen Ligandenbindungsrezeptor) oder ein Fragment davon, einschließlich der Bindungsstelle für das Abfangmittel, zu umfassen, oder wobei der Ligand oder das Ligandenanalog modifiziert ist mit einer bioaktiven Substanz oder einem Derivat oder Fragment davon, die (das) in der Lage ist, sich an das Abfangmittel zu binden.

9. Eine Zusammensetzung nach Anspruch 2 oder 4, wobei das Abfangmittel einen Antikörper oder einen zellulären Rezeptor (verschieden vom spezifischen Ligandenbindungsrezeptor) oder ein Fragment davon, einschließlich der Bindungsstelle für die bioaktive Substanz, umfaßt.

10. Eine Zusammensetzung nach Anspruch 7, wobei das Abfangmittel umfaßt
(a) einen bispezifischen Antikörper,
(b) ein Konjugat eines Antikörpers und eines zellulären Rezeptors (verschieden vom spezifischen Ligandenbindungsrezeptor) oder eines Fragmentes davon, umfassend die Bindungsstelle für die bioaktive Substanz, eines einzelsträngigen Oligonukleotids, eines interzellulären Adhäsionsmoleküls oder eines Komplexierungsmittels (z.B. Biotin, Avidin, Lectin oder ein Medikament) oder
(c) ein Konjugat eines zellulären Rezeptors (verschieden vom spezifischen Ligandenbindungsrezeptor) oder eines Fragmentes davon, umfassend die Bindungsstelle für die bioaktive Substanz, und eines einzelsträngigen Oligonukleotids, eines interzellulären Adhäsionsmoleküls oder eines Komplexierungsmittels (z.B. Biotin, Avidin, Lectin oder ein Medikament).

11. Eine Zusammensetzung nach Anspruch 10, wobei der Ligand oder das Ligandenanalog modifiziert ist, um einen Antikörper, einen zellulären Rezeptor, (verschieden vom Ligandenbindungsrezeptor) oder ein Fragment davon, umfassend eine Bindungsstelle für das Abfangmittel, ein einzelsträngiges Oligonukleotid, ein interzelluläres Adhäsionsmolekül oder ein Komplexierungsmittel zu umfassen, von denen jedes in der Lage ist, ein Konjugat mit dem Teil des Abfangmittels zu bilden, der nicht die bioaktive Substanz bindet.

12. Eine Zusammensetzung nach einem der Ansprüche 1 bis 11, wobei das Abfangmittel mit einer Markierung versehen ist.

13. Eine Zusammensetzung nach Anspruch 12, wobei die Markierung ausgewählt ist aus der Gruppe, die aus radioaktiven Isotopen, Magnetit-Teilchen und paramagnetischen Atomen besteht.

14. Eine Zusammensetzung nach einem der Ansprüche 1 bis 13, wobei der zelluläre Rezeptor ein Leber-, Milz-, Knochenmark-, Lungen-, Nieren-, Haut- oder Plazenta-Rezeptor ist.

15. Eine Zusammensetzung nach einem der Ansprüche 1 bis 14, wobei die bioaktive Substanz ein Medikament (einschließlich eines Narkosemedikamentes), z.B. ein Alkaloid, wie etwa Digoxin oder Morphin, oder ein Antidepressivum (einschließlich tricyclischen Antidepressiva), Acetylsalicylsäure, Paracetamol, Antiepileptikum, β-Rezeptorenblocker, Neuroleptikum, Sympatomimetikum, ein Toxin, z.B. ein Exotoxin oder Endotoxin (LPS), Schlangengift, Pilzgift (z.B. Muscarin), ein anorganisches Gift, wie etwa ein Schwermetall (z.B. Blei, Cadmium, Quecksilber), ein organisches Gift, wie etwa ein Alkaloid (z.B. Nicotin, Scopolamin, Atropin), ein tumorassoziiertes Antigen, fakultativ wie vorhanden auf neoplastischen Zellen, eine von einer neoplastischen Zelle produzierte Substanz, ein aktivierter Leukocyt, ein aktivierter Lymphocyt, eine Plasmazelle, Oberflächen- oder Kreislaufsubstanzen, die von Zellen produziert werden, ein Antikörper, insbesondere ein Auto-Antikörper, ein Thrombus (oder ein Bestandteil davon) oder ein Pathogen (z.B. Virus, Bakterien, Pilze) oder ein Bestandteil davon, wie etwa ein Oberflächenantigen, ist.

16. Eine Zusammensetzung nach einem der Ansprüche 1 bis 15, wobei der Ligand oder das Ligandenanalog ein Wachstumsfaktor, ein Peptid- oder Polypeptidhormon, ein Cytokin, ein enttoxifiziertes Toxin, ein Vitamin, ein Steroidhormon, eine Substanz, die auf das zentrale Nervensystem wirkt, Hyaluronan, Chondroitinsulfat, N-terminales Peptid von Collagen Typ III, Glykokonjugate mit endständiger Mannose, ein Glykosid (gekuppelt an eine bioaktive Substanz) oder Gewebeplasminogenaktivator ist.

17. Verwendung eines Abfangmittels oder eines Liganden oder Ligandenanalogs zur Herstellung einer pharmazeutischen Zusammensetzung, die die schnelle Ausscheidung einer bioaktiven Substanz aus dem Kreislauf bereitstellt und umfaßt
(a) ein Abfangmittel, das in der Lage ist, sich an eine bioaktive Substanz zu binden, um es möglich zu machen, die bioaktive Substanz bei Verabreichung des Abfangmittels zu lokalisieren, zu neutralisieren und/oder zu entfernen; und
(b) einen Liganden oder ein Ligandenanalog, der (das) in der Lage ist, sich an einen zellulären Rezeptor zu binden, der in einem Organ anzutreffen ist, durch das Abfallprodukte des Körpers üblicherweise ausgeschieden werden,
wobei das Abfangmittel mit Mitteln zur Bindung des Liganden oder des Ligandenanalogs versehen ist, oder der Ligand oder das Ligandenanalog mit Mitteln zur Bindung des Abfangmittels versehen ist, oder sowohl das Abfangmittel als auch der Ligand oder das Ligandenanalog mit komplementären Bindungsmitteln versehen sind,
wobei die Zusammensetzung an getrennte und sequentielle Verabreichung des Abfangmittels und des Liganden oder Ligandenanalogs angepaßt ist.

18. Verwendung eines Abfangmittels und eines Liganden oder Ligandenanalogs zur Herstellung einer diagnostischen Zusammensetzung, die die schnelle Ausscheidung des Abfangmittels aus dem Kreislauf und damit einen schnellen Nachweis des fraglichen pathologischen Zustands bereitstellt, wobei die Zusammensetzung umfaßt
(a) ein Abfangmittel, das in der Lage ist, sich an eine bioaktive Substanz zu binden, um es möglich zu machen, die bioaktive Substanz bei Verabreichung des Abfangmittels zu lokalisieren, zu neutralisieren und/oder zu entfernen; und
(b) einen Liganden oder ein Ligandenanalog, der (das) in der Lage ist, sich an einen zellulären Rezeptor zu binden, der in einem Organ anzutreffen ist, durch das Abfallprodukte des Körpers üblicherweise ausgeschieden werden,
wobei das Abfangmittel mit Mitteln, zur Bindung des Liganden oder des Ligandenanalogs versehen ist oder der Ligand oder das Ligandenanalog mit Mitteln zur Bindung des Abfangmittels versehen ist, oder sowohl das Abfangmittel als auch der Ligand oder das Ligandenanalog mit komplementären Bindungsmitteln versehen ist,
wobei die Zusammensetzung an separate und sequentielle Verabreichung des Abfangmittels und des Liganden oder Ligandenanalogs angepaßt ist.

## Revendications

1. Composition pharmaceutique ou de diagnostic comprenant, en récipients séparés,
(a) un agent de capture apte à se fixer sur une substance bioactive de façon à permettre la localisation, la neutralisation et/ou l'élimination de la substance bioactive lors de l'administration de l'agent de capture ; et
(b) un ligand ou analogue ligand capable de se fixer sur un récepteur cellulaire situé dans un organe par lequel sont normalement éliminés les déchets du corps,
l'agent de capture étant muni de moyens pour la fixation du ligand ou de l'analogue ligand, ou le ligand ou l'analogue ligand est muni de moyens pour la fixation sur l'agent de capture, ou aussi bien l'agent de capture et le ligand ou l'analogue ligand sont munis de moyens de fixation complémentaires.

2. Composition selon la revendication 1, dans laquelle l'agent de capture est un agent devant être éliminé rapidement de la circulation.

3. Composition selon la revendication 2, dans laquelle le ligand ou l'analogue ligand est modifié de façon à comprendre un anticorps (comprenant un anticorps anti-idiotype) apte à se fixer sur l'agent de capture, un récepteur cellulaire (autre que le récepteur fixation-ligand spécifique) ou son fragment y compris le site de fixation de l'agent de capture, ou dans laquelle le ligand ou l'analogue ligand est modifié par une substance bioactive ou un dérivé ou son fragment apte à se fixer sur l'agent de capture.

4. Composition selon la revendication 2, dans laquelle la substance bioactive et le ligand sont sensiblement identiques.

5. Composition selon la revendication 1, dans laquelle la substance bioactive est une substance devant être éliminée rapidement de la circulation.

6. Composition selon la revendication 5, dans laquelle la substance bioactive est différente du ligand ou de l'analogue ligand qui est modifié de façon à comprendre un anticorps (y compris un anticorps anti-idiotype) capable de se fixer sur l'agent de capture, un récepteur cellulaire (autre que le récepteur de fixation-ligand spécifique) ou son fragment y compris le site de fixation de l'agent de capture, ou dans lequel le ligand ou l'analogue ligand est modifié par une substance bioactive ou un dérivé ou son fragment apte à se fixer sur l'agent de capture.

7. Composition selon la revendication 5, dans laquelle la substance bioactive est différente du ligand ou de l'analogue ligand.

8. Composition selon la revendication 7, dans laquelle le ligand ou l'analogue ligand est modifié de façon à comprendre un anticorps (y compris un anticorps antiidiotype) apte à se fixer sur l'agent de capture, un récepteur cellulaire (autre que le récepteur fixation-ligand spécifique) ou son fragment y compris le site de fixation de l'agent de capture, ou dans laquelle le ligand ou l'analogue ligand est modifié par une substance bioactive ou un dérivé ou son fragment capable de se fixer sur l'agent de capture.

9. Composition selon la revendication 2 ou 4, dans laquelle l'agent de capture comprend un anticorps ou un récepteur cellulaire (autre que le récepteur fixation-ligand spécifique) ou son fragment y compris le site de fixation de la substance bioactive.

10. Composition selon la revendication 7, dans laquelle l'agent de capture comprend
(a) un anticorps bispécifique,
(b) un conjugué d'un anticorps et d'un récepteur cellulaire (autre que le récepteur de fixation-ligand spécifique) ou son fragment comprenant le site de fixation de la substance bioactive, ou un oligonucléotide monobrin, une molécule d'adhérence intercellulaire, ou un agent complexant (par exemple la biotine, l'avidine, la lectine ou un médicament), ou
(c) un conjugué d'un récepteur cellulaire (autre que le récepteur de fixation-ligand spécifique) ou son fragment comprenant le site de fixation de la substance bioactive et un oligonucléotide monobrin, une molécule d'adhérence intercellulaire, ou un agent complexant (par exemple la biotine, l'avidine, la lectine ou un médicament).

11. Composition selon la revendication 10, dans laquelle le ligand ou l'analogue ligand est modifié de façon à comprendre un anticorps, un récepteur cellulaire (autre que le récepteur de fixation-ligand) ou son fragment comprenant un site de fixation pour l'agent de capture, un oligonucléotide monobrin, une molécule d'adhérence intercellulaire ou un agent complexant, chacun étant apte à former un conjugué avec la partie de l'agent de capture qui ne se fixe pas sur la substance bioactive.

12. Composition selon l'une quelconque des revendications 1-11, dans laquelle l'agent de capture est muni d'un marqueur.

13. Composition selon la revendication 12, dans laquelle le marqueur est choisi dans le groupe constitué par les isotopes radioactifs, les particules de magnétite et les atomes paramagnétiques.

14. Composition selon l'une quelconque des revendications 1-13, dans laquelle le récepteur cellulaire est un récepteur du foie, de la rate, de la moelle osseuse, des poumons, des reins, de la peau ou du placenta.

15. Composition selon l'une quelconque des revendications 1-14, dans laquelle la substance bioactive est un médicament (y compris un produit narcotique), par exemple un alcaloïde tel que la digoxine ou la morphine, ou un antidépresseur (y compris les antidépresseurs tricycliques), l'acide acétylsalicylique, paracétamol, un anti-épileptique, un récepteur β-bloquant, un neuroleptique, un sympatomimétique, une toxine, par exemple une exotoxine ou une endotoxine (LPS), du venin de serpent, du poison fongique (par exemple la muscarine), un poison inorganique tel qu'un métal lourd (par exemple le plomb, le cadmium, le mercure), un poison organique tel qu'un alcaloïde (par exemple la nicotine, la scopolamine, l'atropine), un antigène associé à une tumeur, facultativement présent sur des cellules néoplasiques, une substance produite par une cellule néoplasique, un leucocyte activé, un lymphocyte activé, une cellule plasmatique, des substances de surface ou en circulation produites par des cellules, un anticorps, en particulier un auto-anticorps, un thrombus (ou son composant) ou un agent pathogène (par exemple un virus, une bactérie, des champignons), ou leur composant, tel qu'un antigène de surface.

16. Composition selon l'une quelconque des revendications 1-15, dans laquelle le ligand ou l'analogue ligand est un facteur de croissance, une hormone polypeptide ou peptide, une cytokine, une toxine détoxifiée, une vitamine, une hormone stéroïde, une substance agissant sur le système nerveux central, un hyaluronan, la chondroïtine, un sulfate, un peptide N-terminal de collagène type III, des glucoconjugués terminés par mannose, un glucoside (accouplé à une substance bioactive) ou un activateur de plasminogène tissulaire.

17. Utilisation d'un agent de capture et d'un ligand ou d'un analogue ligand pour la fabrication d'une composition pharmaceutique assurant l'élimination rapide d'une substance bioactive de la circulation et comprenant
(a) un agent de capture capable de se fixer sur une substance bioactive de façon à permettre la localisation, la neutralisation et/ou l'élimination de la substance bioactive lors de l'administration de l'agent de capture ; et
(b) un ligand ou analogue ligand apte à se fixer sur un récepteur cellulaire situé dans un organe par lequel les déchets du corps sont normalement éliminés,
l'agent de capture étant muni de moyens pour se fixer sur le ligand ou l'analogue ligand, ou le ligand ou l'analogue ligand étant munis de moyens pour se fixer sur l'agent de capture, ou à la fois l'agent de capture et le ligand ou l'analogue ligand sont munis de moyens de fixation complémentaires,
la composition pouvant être adaptée à l'administration séparée ou séquentielle de l'agent de capture et du ligand ou analogue ligand.

18. Utilisation d'un agent de capture et d'un ligand ou analogue ligand pour la fabrication d'une composition de diagnostic assurant l'élimination rapide de l'agent de capture de la circulation et par conséquent une détection rapide de la condition pathologique en question, la composition comprenant
(a) un agent de capture capable de se fixer sur une substance bioactive de façon à permettre la localisation, la neutralisation et/ou l'élimination de la substance bioactive lors de l'administration de l'agent de capture ; et
(b) un ligand ou un analogue ligand pouvant se fixer sur un récepteur cellulaire situé dans un organe par lequel les déchets du corps sont habituellement éliminés,
l'agent de capture étant muni de moyens pour se fixer sur le ligand ou l'analogue ligand, ou le ligand ou l'analogue ligand étant munis de moyens pour se fixer sur l'agent de capture, ou aussi bien l'agent de capture que le ligand ou analogue ligand étant munis de moyens de fixation complémentaires,
la composition étant adaptée pour l'administration séparée et séquentielle de l'agent de capture et du ligand ou de l'analogue ligand.
